# EUROPEAN PATENT APPLICATION

(11) **EP 1 223 183 A1**
(43) Date of publication of application: **17.07.2002**
(21) Application number: 02000360.4
(22) Date of filing: 04.01.2002
(51) Int. Cl.: C08G 18/12, C08G 18/10, C08G 18/20, A61F 5/058, A47G 9/00

(54) **Forming material body including granulated materials each coated with a moisture-curable urethane prepolymer**

(30) Priority: 05.01.2001 JP 2001000477
(71) Applicant: Alcare Co., Ltd., Sumida-ku, Tokyo (JP)
(72) Inventor: Matsumoto, Yoshikazu, Funabashi-shi, Chiba (JP); Hirano, Hiroyuki, Funabshi-shi, Chiba (JP)
(74) Representative: Preissner, Nicolaus, Dipl.-Ing.

(57) **Abstract**

This invention provides a forming material which is cured within a short period by a small amount of water, has a molding property necessary for forming and has a sufficient fixing function. In a forming material 4 where a granulated material 1 coated with a moisture-curable urethane prepolymer 2 is tightly sealed with a water-permeable material 3 having smaller openings than the size of the granulated material, the moisture-curable urethane prepolymer 2 is a polyurethane prepolymer comprising an isocyanate and a polyol and the CO % contained therein is made 1-5 % by weight.

## Description

The present invention relates to a forming material for use in the fields of medical treatment, patient care and welfare, sports, etc. for contour or profile modeling parts of human bodies, which thus formed models can be used for the fixation, support, protection and correction of the human bodies.

Various outfits and equipment have been used in the fields of medical treatment and patient care and welfare for fixing and supporting human bodies on beds, chairs, inspection and examination devices and the like. With regard to a forming material by which such various outfits and equipment for fixing, supporting, correction, protection, etc. of human bodies can be easily manufactured, there has been proposed a forming material by the present applicant where a predetermined amount of granulated materials coated with a moisture curable urethane prepolymer is enclosed in a water-permeable container having smaller openings than the size of the granulated materials and the water-permeable material is tightly sealed with a moisture-impermeable container (USP6,027,777 and 6,254,959). In using such a forming material, the moisture-impermeable container is opened to take out the water-permeable container, the water-permeable container is dipped for 5-10 seconds in a water tank filled with water at 15 to 25°C and pulled out from the water tank, an excessive water is removed therefrom, human body part to be fixed or supported is placed on the water-permeable container containing the granulated materials so that the container is made without incongruity to human body part and the human body part is removed after 5-10 minutes followed by drying to give a fixing or supporting device having a curved surface which entirely coincides with the human body part.

Although the above-mentioned forming material has advantages that it can be preserved in a moisture-impermeable material for long time and can be easily handled, a considerable amount of water is needed for curing since it is to be dipped in a water tank. This forming material is quite useful in fixing the tumor site of the object to be irradiated in the case of using radioactive ray in cancer therapy and is often utilized in fixing the patients. However, there is a high risk of danger of affecting the instruments when a lot of water is taken into a chamber, etc. especially for irradiation of radioactive ray where many precise instruments are placed. There is another method where a small amount of water is sprayed onto the forming material instead of a large amount of water is used. However, when this water spraying method is used in the conventional moisture-curable urethane prepolymer, although curing to some extent is achieved, granulated particles are not bonded each other but fall down in pieces whereby a function as a fixing device is not achieved.

It is therefore a principal object of the present invention to provide a forming material which is cured within a short period by the use of a small amount of water giving a molding property necessary for shape forming and a sufficient fixing function.
In order to achieve the above-mentioned object, the present invention is that, in a forming material where granulated materials coated with a moisture-curable urethane prepolymer are tightly sealed with a water-permeable material having smaller openings than the size of the granulated materials, the moisture-curable urethane prepolymer is a polyurethane prepolymer comprising an isocyanate and a polyol and the CO % contained therein is made 1-5 % by weight.

In the present invention, it is advantageous that the moisture-curable urethane prepolymer contains 1-10 % by weight of a catalyst of a morpholinoethyl ether type catalyst.

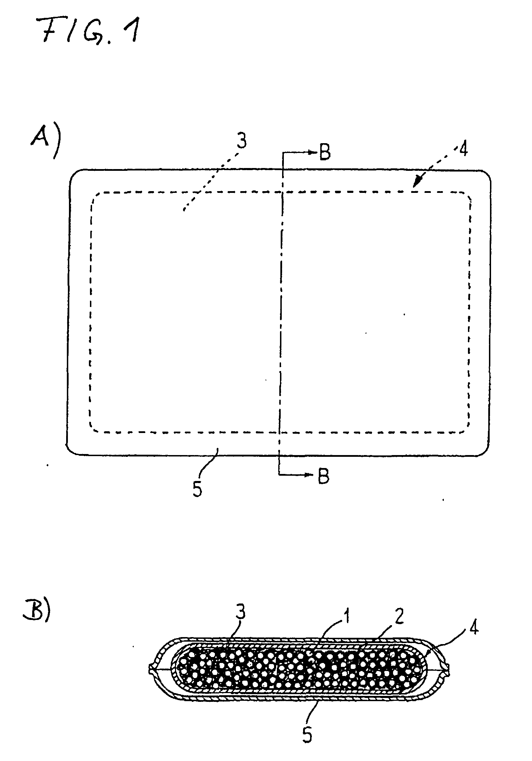

Fig. 1A is a front view showing an embodiment of the present invention and Fig. 1 B is a cross sectional view thereof along the line B-B in Fig. 1A.

The granulated materials used in the present invention are preferably those having an appropriate elasticity and being nonreactive with un-cured moisture-curable resin and the examples are olefin granulated materials to which elasticity is given by a softner or plasticizer such as polyethylene, polypropylene and copolymer thereof; and granulated materials formed from a material to which elasticity is given similarly or which has elasticity such as vinyl acetate copolymer, polyvinyl chloride, polystyrene, polyester, polyether, polyurethane, chloroprene rubber, polybutadiene, silicone and other rubber type materials. It is also possible to use elastic material and gel-like material which is not reactive with the moisture-curable resin.

The granulated material may be formed in various shapes such as sphere, rod, cube, rectangular solid, column and disk and that having no corner is preferred. The granulated material may be either solid or hollow or may be formed into a foamed material. In the case of a foamed material, it is preferred to be a closed cell so that the moisture-curable resin used does not permeate into the granulated material and the material having a skin on the surface is particularly preferred. Although the size of the granulated material varies depending upon the site used, type of the granulated material used and type of the moisture-curable resin, it is preferred to be about 8 cm³ or less and, especially preferably, it is made 0.125 cm³ or less whereby the handling is easy and a fixing device having more smooth surface can be prepared. When the size exceeds 8 cm³, a high air permeability can be ensured but such a material may be inferior in term of shape adapting property and smoothness of the surface. It is also possible that two or more materials having different raw materials, shapes, sizes, etc. are mixed and used.

Hardness of the granulated materials is made about 2 kg/cm² or less or, preferably, about 1 kg/cm² or less in terms of a 25 % compressive hardness according to the hardness test (JIS K 64015.4) of soft urethane foam for cushion. The compressive residual strain (JIS K 64015.5) at this time is about 15 % or less or, preferably, about 13 % or less. When the compressive hardness exceeds 2 kg/cm² or when the residual strain exceeds 15 %, the materials are too hard or result in strain during the use and the usability is not good.

As to the moisture-curable urethane prepolymer coated on the granulated materials, there may be used a prepolymer having an isocyanate group at the terminal obtained by the reaction of the polyol with the polyisocyanate may be used. As to the polyol, there may be used a low-molecular polyol type such as polyethylene glycol, polypropylene glycol and polyglycerol; a polyether polyol type prepared by addition of alkylene oxide such as ethylene oxide or propylene oxide to polyphenol; a polyester polyol type prepared by a dehydrating condensation of low-molecular polyol with dicarboxylic acid such as adipic acid or phthalic acid; a polytetramethylene glycol type prepared by a ring-opening- polymerization of lactone type such as γ-butyrolactone or ε-caprolactone; a polydiene polyol having a hydroxyl group at the terminal which is a polymer of diene compound such as butadiene and isoprene; etc. either solely or jointly. The use of a polyethylene glycol or a polypropylene glycol is particularly advantageous.

An average molecular weight of the polyol in the moisture-curable urethane prepolymer may be made to an extent of about 1,000-6,000 or, preferably, about 1,500-4,000. As to the polyols used at that time, those having the same molecular weights may be used or those having different molecular weights may be mixed and used and, when they are used after mixing, that having a molecular weight of out of the above range may be mixed therewith. To sum up, it is acceptable if the average molecular weight is within the said range. When the molecular weight is small, elasticity becomes poor while, when it is large, elasticity is all right but viscosity becomes high whereby mixing with the granulated materials is difficult. Viscosity at 20°C is about 1-200 Pa.s or, preferably, about 5-100 Pa.s.

As to the polyisocyanate, known organic polyisocyanate may be used and its examples are diphenylmethane diisocyanate, toluene diisocyanate, 1,5-naphthalene diisocyanate, tolidine diisocyanate, hexamethylene diisocyanate, isophorone diisocyanate, p-phenylene diisocyanate, trans-cyclohexane 1,4-diisocyanate, xylene diisocyanate, hydrogenated xylene diisocyanate, hydrogenated diphenylmethane diisocyanate, lysine diisocyanate, triphenylmethane triisocyanate, tris (isocyanate phenyl) thiophosphate, tetramethylxylene diisocyanate, lysine ester triisocyanate, 1,6,11-undecane triisocyanate, 1,8-diisocyanate-4-isocyanate methyloctane, 1,3,6-hexamethylene triisocyanate, bicycloheptane triisocyanate, trimethylhexamethylene diisocyanate, polymethylenepolyphenylene polyisocyanate, 3-isocyanate methyl 3,5,5-trimethylcyclohexyl isocyanate, a carbodiimide modified polyisocyanate thereof and an isocyanurate modified polyisocyanate thereof. Each of those polyisocyanates may be used solely or two or more thereof may be used jointly. It is particularly advantageous to use an aromatic polyisocyanate such as diphenylmethane diisocyanate, p-phenylene diisocyanate and polymethylene polyphenylene polyisocyanate as well as carbodiimide-modified polyisocyanate thereof.

The compounding ratio of the polyol to the polyisocyanate for preparing a urethane prepolymer having an isocyanate group at the terminal varies depending upon the polyol and the polyisocyanate used therefor and they may be compounded so as to make the amount of CO group therein 1-5 % by weight. Especially when the compounding is carried out to make the CO amount 2-4 % by weight, water amount necessary for curing of the resulting prepolymer and molding property upon application are good. When the CO amount is less than 1 % by weight, curing is difficult in the reaction of urethane prepolymer with water and, even when a long period elapses, curing does not proceed and the product cannot be used as a forming material. On the other hand, when the CO amount is more than 5 % by weight, curing by the reaction of urethane prepolymer with water proceeds in a tattered state and it is not possible to bond the granular materials whereby the product is unable to be used as a forming material.

A catalyst, a stabilizer, an antifoaming agent, an antioxidant, a coloring agent, a thixotropic agent, etc. may be added to a moisture-curable resin by taking curing time, stability in storage, promotion of defoaming during curing, color tone upon finish, etc. into consideration. As to those additives, known compounds corresponding to the components of the moisture-curable resin may be appropriately used.

As to the catalyst, various ones which have been used for the moisture-curable resin may be used. The use of a catalyst of a morpholinoethyl ether type is particularly advantageous. Those examples are bis(2,6-dimethylmorpholino) diethyl ether and dimorpholino diethyl ether. It is preferred that the catalyst is used in an amount of about 1-10 % by weight to the urethane prepolymer. When the amount of the catalyst is less than 1 % by weight such as 0.5 % by weight, no curing takes place even when 30 minutes elapse and that is not practical in view of the curing time. On the other hand, when it is more than 10 % by weight such as 15 % by weight, that is not so effective in view of curing property or, rather, an increase in the catalyst amount acts as a plasticizer whereby that acts for lowering the strength of the resin itself and makes the stability on storage bad.

As to the stabilizer, there are acidic substances such as organic acid, organic acid chloride and acidic phosphate ester or the like, chelating agent (such as diketone compound, hydroxycarboxylic acid) and an appropriate one in view of combination with the catalyst may be used. For example, an organic acid of a toluenesulfonic acid type is suitable. Adding amount is usually about 0.01-3 % by weight.

As to the antifoaming agent, it is preferred to use that of a silicon type in an amount of about 0.01-2 % by weight. As to the antioxidant, hindered phenol, phosphoric compound, etc. are available. As to the coloring agent, it is preferred to use an official coloring matter (the coloring matter by the Japanese Pharmaceutical Affairs Law) having little danger of dermatitis. When a thixotropic agent is used, uneven disposition of the water-curable resin during the storage in a mixed state of the water-curable resin and the granulated material can be prevented whereby it is possible to maintain a state where both are uniformly mixed. As to the thixotropic agent, there may be used silica; titanium oxide; a polyalkylene modified compound obtained by treating the terminal hydroxyl group of an organic polyalkylene glycol with a hydroxyl group treating agent wherein, for example, polyethylene glycol, polypropylene glycol or a copolymer thereof is treated with methyl chloride, fatty acid or the like; an aromatic carboxylate; benzylidene sorbitol, ditolylidene sorbitol, etc. synthesized by an acetal reaction of D-sorbitol with an aromatic aldehyde; etc. The using amount is about 0.01-6 % by weight or, preferably, about 0.05-3 % by weight to the water-curable urethane prepolymer.

Mixing amount of the moisture-curable urethane prepolymer to the granulated material per 1 liter of the granulated material is about 7.5 g or more or, preferably, about 45-420 g. Mixing of the granulated material with the moisture-curable urethane prepolymer is carried out in such a manner that, under the circumstance of 20 °C and relative humidity of 20 % or less, a mixer is used, dry nitrogen gas is filled in a mixing container, a predetermined amount of the granulated material is poured thereinto and a predetermined amount of the moisture-curable urethane prepolymer is added thereto with stirring followed by mixing until they become uniform. In a mixture of the granulated material with the moisture-curable urethane prepolymer prepared as such, surface of the granular material is covered by the moisture-curable urethane prepolymer in an amount sufficient for connecting the granulated materials each other and the granulated materials are bonded each other by the moisture-curable urethane prepolymer and are kept in a movable state each other.

It is preferable to ensure that the granulated materials with their surfaces covered by a moisture-curable polyurethane prepolymer are put, or wrapped, in a predetermined amount in conformity with the appliance or equipment to be formed, into a bag-like container composed of a water-permeable material and to keep the bag in a moisture-impermeable container or vessel in a hermetically sealed state until the forming material is put to actual use.

The water-permeable material wrapping up or containing the granulated materials therein facilitates putting together the predetermined amount of granulated materials coated with a moisture-curable urethane prepolymer into one mass and isolating the granulated materials so that the operator and the appliance wearer or user can handle the forming material without directly touching the urethane prepolymer to thereby avoid the adverse effect on their skin by the resin and also to facilitate the operation. Since it is desired that the water-permeable material is unreactive with the resin and the resin is a moisture-curable urethane prepolymer, the water-permeable material should be a material which has a low water content; that is, a material which does not contain a material or substance having a chemical structure which activates the reactive group of the urethane prepolymer. As for the size,shape and structural properties of the water-permeable material container, it is important, in improving the maneuverability or operability, to select a water permeable container having properties in conformity with the appliance or equipment to which the forming material is applied.

As to a raw material for the water-permeable material, there may be used synthetic fibers such as polyester, polypropylene, polyethylene, polyacrylate, polyurethane, styrene-isoprene-styrene copolymer (SIS) and polyamide etc.; regenerated fibers such as rayon, staple fiber, natural fibers such as cotton and linen; inorganic fibers such as glass fiber; etc. and, in the case having a reactivity with the moisture-curable urethane prepolymer or having a high in water content, it is recommended that the surface is previously treated to make it nonreactive or dried up to remove water therefrom. Examples of a preferred raw material for the moisture-curable urethane prepolymer are polyester, polypropylene, polyethylene, polyurethane, and SIS which do not react with the un-cured urethane prepolymer. Particularly preferred ones are polyester, polypropylene, polyethylene, polyurethane and SIS having a heat sealing property as well as a fiber where any of the above is subjected to a mix spinning.

The water-permeable material is formed into a knitted fabric, an woven fabric, a nonwoven fabric, a melted net, etc. and it is preferred to give such a form that flexibility and stretchability for getting to fit the irregular shape of the applied site are easily available in actual use. To obtain appropriate flexibility and stretchability, the fabric which is used preferably should have an elongation of about 15 % or more in at least one of the lengthwise and breadthwise directions. If the elongation is less than that, it may be difficult for modeling in forming the shape.

As to the water-permeable material, a material having a low affinity with the moisture-curable urethane prepolymer as mentioned above is preferred and its adhesive strength with the granular material and the moisture-curable urethane prepolymer enclosed therein is preferably about 0.5 kg/25 mm or less (an adhesive strength according to JIS Z 0237.8). If the adhesive strength is more than that, the moisture-curable resin and the bag may be brought into one integral body during a long-term preservation whereby the material may become unusable. When the actual operability is taken into consideration, the above adhesive strength is preferably about 0.3 kg/25 mm or less and, more preferably, about 0.1 kg/25 mm or less.

When a knitted fabric or an woven fabric is used as the water-permeable material, it may be formed by yarns where many thin fibers are aggregated but, in such a case, it is preferred that a treatment for making the affinity with the moisture-curable urethane prepolymer low is carried out so that the moisture-curable urethane prepolymer does not penetrate into the thin fibers. Such a treatment may be carried out by a treating agent of a fluorine series, a silicon series , a paraffin series, an alkyl chromic chloride series, an alkyl ethylene urea series, an alkylmethylpyridium chloride series, etc. The treating agent is used in such an amount that its effective component adheres in about 0.1-6 % by weight and the treating agent may be adhered by means of impregnation, application, spraying, etc. before or after the production of the knitted fabric, the woven fabric, etc.

A preferred example of the water-permeable material is a knitted fabric comprising yarns of about 200 deniers made of one of or both polypropylene and polyester being finished in a tube-like shape with about 22 yarns/inch in the courses direction and about 22 yarns/inch in the wales direction and having a weight per unit area of about 230 g where elongations in the longitudinal direction and the lateral direction are 5-60 % and 50-300 %, respectively. For example, "White Net" (trade name; manufactured by Alcare Co., Ltd.) may be used. When a treating agent of a fluorine series emulsion is adhered onto the surface of the water-permeable material to make the amount of the effective component 0.7 % by weight, the storage stability becomes higher and the operability becomes easier.

When a mixture of the granulated material and the moisture-curable urethane prepolymer is received in a bag comprising a water-permeable material and the bag is sealed in a moisture-impermeable container such as that made of aluminum foil until its use, it is possible to prevent a phenomenon that, during the storage, the content is cured by moisture in the air and becomes unusable.

### Examples

Now, examples of the present invention will be illustrated as hereunder. Thus, examples of the moisture-curable urethane prepolymer were prepared by mixing the compounding materials as mentioned hereinafter.

### Example 1

| | |
|---|---|
| Polyol component (PPG-2000) | 750 g |
| Polyisocyanate component (Isonate 125 M) | 184 g |
| Catalyst (UCAT 660 M) | 60 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |
| (Notes) PPG-2000: polypropylene glycol having an average molecular weight of 2,000 (manufactured by Sanyo Chemical Industries, Ltd.) Isonate 125 M: 4,4-diphenylmethane diisocyanate (manufactured by Mitsubishi Chemical-Dow Corporation) UCAT 660 M: morpholino ethyl ether (manufactured by Sanyo Chemical Industries, Ltd.) BYK-A 525: antifoaming agent of a silicone type (manufactured by BYK Chemie) Irganox 1010: antioxidant of a hindered phenol type (manufactured by Nagase-CIBA) Gelol D: thixotropic agent (manufactured by New Japan Chemical Co., Ltd.) | |

In this moisture-curable urethane prepolymer, content of CO was 3.3 % by weight, content of the catalyst was 5.9 % by weight and viscosity was 30.4 Pa·s.

### Example 2

| | |
|---|---|
| Polyol component (PPG-2000) | 750 g |
| Polyisocyanate component (Isonate 125 M) | 215 g |
| Catalyst (UCAT 660 M) | 65 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 4.0 % by weight, content of the catalyst was 6.3 % by weight and viscosity was 29.2 Pa·s.

### Example 3

| | |
|---|---|
| Polyol component (PPG-2000) | 750 g |
| Polyisocyanate component (Isonate 125 M) | 230 g |
| Catalyst (UCAT 660 M) | 70 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 4.5 % by weight, content of the catalyst was 6.6 % by weight and viscosity was 28.6 Pa·s.

### Example 4

| | |
|---|---|
| Polyol component (PPG-1000) | 740 g |
| Polyisocyanate component (Isonate 125 M) | 260 g |
| Catalyst (UCAT 660 M) | 60 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

### (Notes)

PPG-1000: polypropylene glycol having an average molecular weight of 1,000 (manufactured by Sanyo Chemical Industries, Ltd.)

In this moisture-curable urethane prepolymer, content of CO was 2.4 % by weight, content of the catalyst was 5.6 % by weight and viscosity was 50 Pa·s.

### Example 5

| | |
|---|---|
| Polyol component (PPG-4000) | 815 g |
| Polyisocyanate component (Isonate 125 M) | 185 g |
| Catalyst (UCAT 660 M) | 60 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

### (Notes)

PPG-4000: polypropylene glycol having an average molecular weight of 4,000 (manufactured by Sanyo Chemical Industries, Ltd.)

In this moisture-curable urethane prepolymer, content of CO was 4.2 % by weight, content of the catalyst was 5.6 % by weight and viscosity was 8 Pa·s.

Now, comparative examples of the moisture-curable urethane prepolymer were prepared by mixing the compounding materials as mentioned hereinafter.

### Comparative Example 1

| | |
|---|---|
| Polyol component (PPG-2000) | 657 g |
| Polyisocyanate component (Isonate 125 M) | 250 g |
| Catalyst (UCAT 660 M) | 60 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 6.0 % by weight, content of the catalyst was 6.2 % by weight and viscosity was 26.6 Pa·s.

### Comparative Example 2

| | |
|---|---|
| Polyol component (PPG-2000) | 657 g |
| Polyisocyanate component (Isonate 125 M) | 321 g |
| Catalyst (UCAT 660 M) | 70 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 8.0 % by weight, content of the catalyst was 6.7 % by weight and viscosity was 24.0 Pa·s.

### Comparative Example 3

| | |
|---|---|
| Polyol component (PPG-2000) | 700 g |
| Polyisocyanate component (Isonate 125 M) | 219 g |
| Catalyst (UCAT 660 M) | 60 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 9.4 % by weight, content of the catalyst was 6.1 % by weight and viscosity was 21.5Pa·s.

### Comparative Example 4

| | |
|---|---|
| Polyol component (PPG-2000) | 657 g |
| Polyisocyanate component (Isonate 125 M) | 321 g |
| Catalyst (UCAT 660 M) | 20 g |
| Stabilizer (p-toluenesulfonic acid monohydrate) | 1.3 g |
| Antifoaming agent (BYK-A 525) | 1 g |
| Antioxidant (Irganox 1010) | 1 g |
| Thixotropic agent (Gelol D) | 0.6 g |

In this moisture-curable urethane prepolymer, content of CO was 8.0 % by weight, content of the catalyst was 1.9 % by weight and viscosity was 24.0 Pa·s.

Then forming materials were prepared using the above-mentioned moisture-curable urethane prepolymers and a comparative test was carried out for their properties. Thus, 45 g of a 30-fold foamed polystyrene beads were uniformly mixed with 91 g of the moisture-curable urethane prepolymer of each of the above Examples and Comparative Examples and wrapped with a water-permeable material (a bag having a size of 20 × 25 cm) which was not reactive with the resin to prepare a test sample and all of them were tightly sealed with a moisture-impermeable material (aluminum foil case). In conducting the test, each test sample was taken out from a tightly sealed moisture-impermeable material, a spray nozzle was contacted the test sample and water was sprayed at nearly the same intervals for 9 times for one side and 18 times for both sides so as to penetrate the water into the inner area. Since about 1 g of water was discharged for one spraying, about 18 g of water were sprayed onto the test sample on both sides but water in such an amount did not cause unpleasant feel to patients. On the contrary, the conventional curing method for a forming material of such a type with water is in such a manner that, in terms of the above-mentioned test sample, it is dipped in water for 5-10 seconds, taken out, squeezed to such an extent that water does not drip down when pushed with both hands (about 10 seconds) and formed into a predetermined shape. In such a means however, residual water amount is about 80 g and such a water amount wetted the patient to be modeled giving unpleasant feel to him/her. The test sample after spraying was immediately placed beneath the diseased part and modeled on the shape of the diseased part and the time until curing and the molding property were measured. The result is as follows.

**Table 1**

| | Example 1 | Example 2 | Example 3 | Example 4 | Example 5 |
|---|---|---|---|---|---|
| Curing Time | 5 min and 30 sec | 5 min and 10 sec | 4 min and 40 sec | 5 min | 6 min and 10 sec |
| Molding property | ○ | ○ | ○ | ○ | ○ |

Curing time was the time until hardness became 90 using an Ascar F hardness tester while, with regard to the molding property, "○" is that when the test sample flexibly followed the diseased part of the patient whereby modeling was possible, "Δ" is that when the test sample was able to model but partial deformation was noted and "×" is that when the test sample showed deformation during the modeling.

**Table 2**

| | Comp. Ex. 1 | Comp. Ex. 2 | Comp. Ex. 3 | Comp. Ex. 4 |
|---|---|---|---|---|
| Curing Time | 5 min and 10 sec | 4 min and 30 sec | 4 min and 30 sec | 10 min or longer |
| Molding Property | Δ | x | x | x |

Meanings for the numerals and the signs for each of curing time and molding property are the same as those for Table 1.

As will be apparent from Tables 1 and 2, all of the cases of Examples of the present invention showed no big change from the conventional products in terms of curing time and the time was sufficient for modeling and was so short that the patient was well able to keep still from molding to curing. Molding property was good as well where the product flexibly followed the applied part and the granulated materials were bonded each other to solidify without resulting in tatters to afford a fixing function precisely corresponding to the shape of the part. On the contrary, in the cases of Comparative Examples, although there was no particular problem for curing time except Comparative Example 4, molding property was not good that, in Comparative Example 1, although the modeling was possible, deformation partly took place during the curing whereby it was not possible to give a shape precisely followed the applied part and there lacked in a practical value and, in Comparative Examples 2-4, deformation took place during the modeling and a shaping to the applied site was impossible.

Mechanism of a water curing of the above-mentioned Examples and Comparative Examples is as follows. For instance, in Example 1, the product has a structure where the isocyanates are added to both terminals of the polyol and the CO contained therein is 3.3 % by weight while, in each of Comparative Examples, free isocyanates are present and the CO contained therein are more than 5 % by weight. Since there are no free isocyanates in the product of Example 1, prepolymers are bonded via a urea bond by a water curing while, in Comparative Examples, free isocyanates are present and, therefore, there are formed urea bonds between prepolymers and isocyanates and between isocyanates as well in addition to the urea bond between prepolymers. Accordingly, in Comparative Examples, many urea bonds and diphenylmethane skeletons are present and the rate of a hard and fragile structure increases. Such a point tends to be prominent when NCO % becomes more than 5 % by weight and still further increases. When the NCO % is lower than 1 % by weight, it is necessary to use a particularly high-molecular polyol whereby viscosity of the urethane prepolymer becomes high and that is not preferred in view of manufacture and use.

Fig. 1A is a front view as a whole of the Examples of the present invention and Fig. 1B is a cross sectional view thereof along the line B-B in Fig. 1A. 1 is a granulated material and it is coated with a moisture-curable urethane prepolymer 2 comprising an isocyanate and a polyol where the NCO % contained therein is within a range of 1-5 % by weight and received in a bag-shaped water-permeable material 3 whereupon a forming material 4 is produced. This forming material 4 is further sealed in a moisture-impermeable material 5 and stored. In its use, the moisture-impermeable material 5 is unsealed, the forming material 4 is taken out, sprayed with an appropriate amount of water using a spray, attached to the diseased part of the body to model and is held until the resin is cured.

In accordance with the present invention, the moisture-curable urethane prepolymer resin can be cured using a very little amount of water obtained by means of spraying within a sufficient time required for modeling and also within a short period. Therefore, various kinds of outfits and equipment having a curved surface precisely corresponding to the various figures of human body can be prepared quite easily within a short period and a forming material which is usable even in a medical treatment room where the use of a lot of water is not preferred.

## Claims

1. In a forming material where a granulated material coated with a moisture-curable urethane prepolymer is tightly sealed with a water-permeable material having smaller openings than the size of the granulated material, a moisture-curable forming material which is **characterized in that** the moisture-curable urethane prepolymer is a polyurethane prepolymer comprising an isocyanate and a polyol and the NCO % contained therein is made 1-5 % by weight.

2. The moisture-curable forming material according to claim 1, wherein the moisture-curable urethane prepolymer contains 1-10 % by weight of catalyst of a morpholino ethyl ether type.

3. The moisture-curable forming material according to claim 1, wherein an average molecular weight of the polyol is 1,000-6,000.

4. The moisture-curable forming material according to claim 1, wherein the granulated material is not reactive with a non-cured moisture-curable urethane prepolymer.

5. The moisture-curable forming material according to claim 1, wherein the granulated material is elastic and has a size of 8 cm³ or less.

6. The moisture-curable forming material according to claim 1, wherein the affinity of the water-permeable material for the moisture-curable urethane prepolymer is little.

7. The moisture-curable forming material according to claim 1, wherein the moisture-curable urethane prepolymer contains a catalyst, a stabilizer, an antifoaming agent and an antioxidant.

8. The moisture-curable forming material according to claim 7, wherein the moisture-curable urethane prepolymer contains a thixotropic agent.

9. The moisture-curable forming material according to claim 1, wherein a hardness of the granulated material is 2 kg/cm² or less in terms of a 25 % compressive hardness and the compressive residual strain at this time is 15 % or less.
